# EUROPEAN PATENT APPLICATION

(11) **EP 1 584 372 A2**
(43) Date of publication of application: **12.10.2005**
(21) Application number: 05007430.1
(22) Date of filing: 05.04.2005
(51) Int. Cl.: B01J 19/00

(54) **A microarray having microarray identification information stored in the form of a spot. Method of producing the microarray and method of using the microarray**

(30) Priority: 10.04.2004 KR 2004024738
(71) Applicant: SAMSUNG ELECTRONICS CO., LTD., Gyeonggi-do (KR)
(72) Inventor: Kwon, Taejoon, Seongnam-si Gyeonggi-do (KR)
(74) Representative: Grünecker, Kinkeldey, Stockmair & Schwanhäusser Anwaltssozietät

(57) **Abstract**

Provided are a microarray having microarray identification information stored in the form of a spot, and a method of producing the microarray. The stored microarray identification information can be read in a convenient and efficient manner.

## Description

### BACKGROUND OF THE INVENTION

### 1. Field of the Invention

The present invention relates to a microarray having microarray identification information stored in the form of a spot, and a method of producing the microarray.

### 2. Description of the Related Art

Microarrays, such as nucleic acid microarrays or protein microarrays, are used in various analytical methods. The analysis methods using microarrays have an advantage in that they can reveal a great deal information in a short time. However, the results obtained by using microarrays should be interpreted differently according to the manufacturing conditions or the characteristics of microarrays. Thus, it is necessary to identify microarray identification information during merchandising and when analyzing the experimental results obtained by using the microarray.

Methods of recording microarray identification information using a bar code are known in the art. For example, U.S. Patent No. 6,399,365 discloses a package for hybridization comprising a housing containing a bar code for microarray identification information. U.S. Patent No. 6,215,894 discloses a microarray having a machine-readable array identifier. However, these methods require a separate reader, apart from a microarray reader generally used to read the results of the experiment obtained in an analytical method using the microarray. That is, microarray identification information should be recorded in a space apart from an array of probes and a separate reader should be used. In addition, the location where the bar code is disposed may depend on microarray manufacturers, and thus, the reader should be manually controlled.

The present inventors conducted a considerable amount of research to overcome these problems and designed a microarray of which identification information can be read using the same reader that is used to detect the results of an experiment in an analytical method using the microarray, without using a separate instrument, such as a bar code, in a separate space apart from a microarray of probes.

### SUMMARY OF THE INVENTION

The present invention provides a microarray of which identification information can be read in a convenient and efficient manner.

The present invention also provides a method of producing a microarray of which identification information can be read in a convenient and efficient manner.

According to an aspect of the present invention, there is provided a microarray having microarray identification information stored in the form of a spot.

According to another aspect of the present invention, there is provided a method of producing a microarray having microarray identification information stored in the form of a spot, comprising: coding the microarray identification information as a number; and immobilizing a detectable substance on a solid substrate, the coded number being expressed by using a combination of a spot having a detectable substance immobilized thereon and a spot not having a detectable substance immobilized thereon.

According to still another aspect of the present invention, there is provided a method of producing a microarray having microarray identification information stored in the form of a spot, comprising: coding the microarray identification information to a number; and having a surface of a solid substrate subjected to a treatment so that a detectable signal can be generated therefrom, the coded number being expressed by using a combination of a spot having a surface subjected to the treatment and a spot having a surface not subjected to the treatment.

### BRIEF DESCRIPTION OF THE DRAWINGS

The above and other features and advantages of the present invention will become more apparent by describing in detail exemplary embodiments thereof with reference to the attached drawings in which:
FIG. 1A is a view illustrating a conventional microarray having microarray identification information stored using a bar code;
FIG. 1B is a view illustrating a microarray according to an embodiment of the present invention having microarray identification information expressed stored spots;
FIG. 2A is a view illustrating a binary encoding table listing spots representing integers between 0 and 9, a discriminator for discriminating an information unit, and an "END" letter designating the end of information;
FIGS. 2B and 2C are views illustrating binary encoding tables litsting spots representing the 26 letters of the Alphabet; and
FIG. 3 is a view illustrating microarray identification information "SAIT-SM30003-1" stored in the upper 1^{st} to 3^{rd} rows of the spot region in a microarray.

### DETAILED DESCRIPTION OF THE INVENTION

An embodiment of the present invention provides a microarray having microarray identification information stored in the form of a spot.

In the present embodiment, the microarray identification information may be stored using a combination of a spot having a detectable substance immobilized thereon and a spot not having a detectable substance immobilized thereon. The detectable substance refers to a substance that generates a detectable signal, for example, by irradiation of light and a reaction of an enzyme with a substrate. Examples of the detectable signal include light, such as fluorescence, phosphorescence, and radioactive rays, and a difference of reflection during irradiation of light. Examples of the detectable substance include those selected from the group consisting of a fluorescent substance, a radioactive substance and an enzyme capable of generating a detectable signal. The detectable substance may generate the same signal as signals indicating a degree of correlation between a probe substance and a target substance. For example, if a target which is a nucleic acid is labeled with a fluorescent dye, such as Cy-3 and Cy-5, the fluorescent dye can be used as a detectable substance. Thus, a detector used for determining the results of a correlation between the probe substance and the target substance can be also used for reading the microarray identification information, which would eliminate a separate detector for reading. In addition, the signal determined and read through the detector can be stored in an extractable storage medium. Therefore, there is no need for separately inputting of the microarray identification information.

In the present embodiment of the present invention, the microarray identification information may be stored in the form of a binary number or a tertiary number. As used herein, the term "microarray identification information" refers to information on a microarray itself, i.e., information on the characteristics, a manufacturing process of a microarray, the manufacturer, and immobilized probes etc. The microarray identification information may be expressed in a language which human can read and understand, such as letters, numbers or symbols. In the present embodiment of the present invention, the microarray identification information may be encoded into numbers and expressed in a binary number or a tertiary number using a spot on a substrate of the microarray.

In the present embodiment of the present invention, one type of the detectable substance may be used and the microarray identification information may be stored on a spot of the microarray in the form of a binary number by representing a signal generated from the spot having the detectable substance immobilized thereon by 0 or 1 and a signal generated from the spot not having the detectable substance immobilized thereon by 1 or 0, in a respective order. In addition, two types of detectable substances may be used and the microarray identification information may be stored on a spot of the microarray in the form of a tertiary number by representing a signal generated from the spot having a first detectable substance immobilized thereon by 0, 1 or 2, a signal generated from the spot having a second detectable substance immobilized thereon by 1, 2 or 0, and a signal generated from the spot not having the first or the second detectable substance immobilized thereon by 2, 0 or 1, in a respective order.

As used herein, the term "microarray" refers to a substrate in which specific molecules are densely immobilized in a predetermined region. Examples of the microarray include, but are not limited to, a polynucleotide microarray and a protein microarray. The term "polynucleotide microarray" refers to a substrate in which polynuleotides are densely immobilized in each predetermined region. The microarray is well known in the art, for example, U.S. Patent Nos. 5,445,934 and 5,744,305. Methods of producing a microarray using photolithography are known in the art. One method includes coating a surface of substrate with a monomer protected with a removable protecting group, exposing a predetermined region of the surface to an energy source to remove the protecting group, and coupling a monomer protected with a removable protecting group to the monomer, and repeating the process, thereby producing a polynucleotide microarray. In this method, a polynucleotide is synthesized by extending monomers one by one, thus allowing immobilization of a polynucleotide. In a method of producing a microarray using spotting, a polynucleotide previously synthesized is immobilized in a predetermined region on a substrate. The method of producing a microarray using spotting is described, for example, in U.S. Patent Nos. 5,744,305, 5,143,854 and 5,424,186. All the above patents are incorporated herein in their entirety by reference.

As used herein, the term "probe substance" refers to a substance which is immobilized on a substrate and specifically binds to a target substance. Examples of a probe substance include nucleic acids, proteins, and sugars. For example, if a target substance is a polynucleotide, a probe substance may be a polynuleotide complementary to the target polynucleotide. If a target substance is a protein, a probe substance may be a ligand, an antigen or an antibody, or the like which specifically binds to the target protein.

As used herein, the term "spot" refers to a region on which the probe substance is immobilized or will be immobilized in rows and columns in a substrate. The spot may include a region on the substrate, on which the probe substance is not immobilized.

In the present embodiment of the present invention, the microarray identification information may be stored using a combination of a spot having a surface subjected to a treatment so that a detectable signal can be generated therefrom and a spot not having a surface subjected to the treatment.

In the present embodiment of the present invention, the microarray identification information is stored in the form of a binary number or a tertiary number.

In the present embodiment of the present invention, one type of surface treatment may be used and the microarray identification information may be stored in the form of a binary number by representing a signal generated from the spot having the surface subjected to the treatment so that the detectable signal can be generated therefrom by 0 or 1 and a signal generated from the spot not having the surface subjected to the treatment by 1 or 0, in a respective order. In addition, two types of surface treatments may be used and the microarray identification information may be stored in the form of a tertiary number by representing a signal generated from the spot having the surface subjected to a first treatment so that the detectable signal can be generated therefrom by 0, 1 or 2, a signal generated from the spot having the surface subjected to a second treatment so that the detectable signal can be generated therefrom by 1, 2 or 0, and a signal generated from the spot not having the surface subjected to the first or the second treatment by 2, 0 or 1, in a respective order.

In the present embodiment of the present invention, the surface treatment may be any treatment that generates a detectable signal. Examples of the surface treatment include a treatment by which the reflection of light can be changed, such as the formation of a grid.

Another embodiment of the present invention provides a method of producing a microarray having microarray identification information stored in the form of a spot, comprising: coding the microarray identification information to a number; and immobilizing a detectable substance on a solid substrate, the coded number being expressed by using a combination of a spot having a detectable substance immobilized thereon and a spot not having a detectable substance immobilized thereon.

In the present embodiment of the present invention, the detectable substance may generate the same signal as those indicating a degree of correlation between a probe and a target. In addition, the microarray identification information may be stored in the form of a binary number or a tertiary number, but are not limited thereto.

In the present embodiment of the present invention, one type of detectable substance may be used and the microarray identification information may be stored in the form of a binary number by representing a signal generated from the spot having the detectable substance immobilized thereon by 0 or 1 and a signal generated from the spot not having the detectable substance immobilized thereon by 1 or 0, in a respective order. In addition, two types of detectable substances may be used and the microarray identification information may be stored in the form of a tertiary number by representing a signal generated from the spot having a first detectable substance immobilized thereon by 0, 1 or 2, a signal generated from the spot having a second detectable substance immobilized thereon by 1, 2 or 0, and a signal generated from the spot not having the first or the second detectable substance immobilized thereon by 2, 0 or 1, in a respective order.

Still another embodiment of the present invention provides a method of producing a microarray having microarray identification information stored in the form of a spot, comprising: coding the microarray identification information to a number; and having a surface of a solid substrate subjected to a treatment so that a detectable signal can be generated therefrom, the coded number being expressed by using a combination of a spot having a surface subjected to the treatment and a spot having a surface not subjected to the treatment.

In the present embodiment of the present invention, the microarray identification information may be stored in the form of a binary number or a tertiary number.

In the present embodiment of the present invention, one type of surface treatment may be used and the microarray identification information may be stored in the form of a binary number by representing a signal generated from the spot having the surface subjected to the treatment so that the detectable signal can be generated therefrom by 0 or 1 and a signal generated from the spot having the surface not subjected to the treatment by 1 or 0, in a respective order. In addition, two types of surface treatments may be used and the microarray identification information may be stored in the form of a tertiary number by representing a signal generated from the spot having the surface subjected to a first treatment so that the detectable signal can be generated therefrom by 0, 1 or 2, a signal generated from the spot having the surface subjected to a second treatment so that the detectable signal can be generated therefrom by 1, 2 or 0, and a signal generated from the spot not having the surface subjected to the first or the second treatment by 2, 0 or 1, in a respective order.

In the present embodiment of the present invention, the surface treatment may be any treatment that generates a detectable signal. Examples of the surface treatment may include a treatment by which the difference of reflection, transmittance or energy level of light, which can be confirmed using a microarray reader, can be generated. Specific examples include, but are not limited thereto, formation of a grid or coating of a substance which causes a change of reflection on a surface.

Yet another embodiment of the present invention provides a method of using a probe microarray, comprising: reacting a target substance with a probe substance immobilized on the microarray having microarray identification information stored in the form of a spot according to a previous embodiment of the present invention and determining a signal generated due to the reaction; and reading the microarray identification information stored in the form of a spot.

In the method of using a probe microarray according to the present embodiment, the determining the signal due to the reacting and the reading the microarray identification information may be performed using an identical detector. Thus, a separate detector is not required in the reading of the microarray identification information.

In the present embodiment of the present invention, the method of using a probe microarray further comprises storing data obtained from the determining the signal due to the reaction and the microarray identification information obtained from the reading, in an extractable storage medium. Thus, the data of the experiment and the microarray identification information can be simultaneously processed, without separately inputting the microarray identification information to a storage medium. The data of the experiment data and the microarray identification information stored in the extractable storage medium can be conveniently used in the following data exchange or operation using a computer, or the like. For example, a researcher may identify information on the manufacturer and the version of a microarray product from the microarray identification information stored in the microarray, and then using the information, request a analytical algorithm suitable for analyzing the product through an Internet site of the manufacturer.

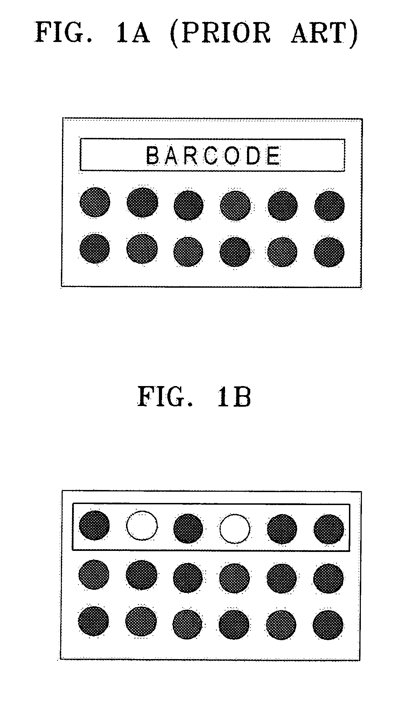

FIG. 1A is a view illustrating a conventional microarray having microarray identification information stored using a bar code and FIG. 1B is a view illustrating a microarray according to an embodiment of the present invention having microarray identification information stored using spots. As illustrated in FIGS. 1A and 1B, while microarray identification information is expressed using a separate bar code for the conventional microarray, it is expressed using spots in the microarray according to an embodiment of the present invention.

Hereinafter, the present invention will be described in more detail by means of the following example. The example is presented for illustrative purposes only and is not intended to restrict the scope of the invention.

### EXAMPLE

In the EXAMPLE, microarray identification information was encoded in a binary number, and then stored in the form of a spot by immobilizing a fluorescent substance on a substrate of a microarray in the same manner as in the process of immobilizing a probe on the substrate.

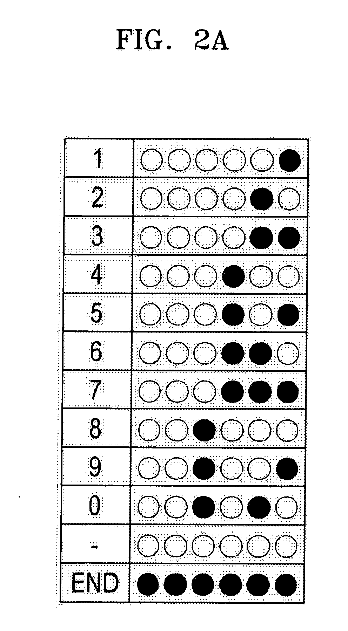

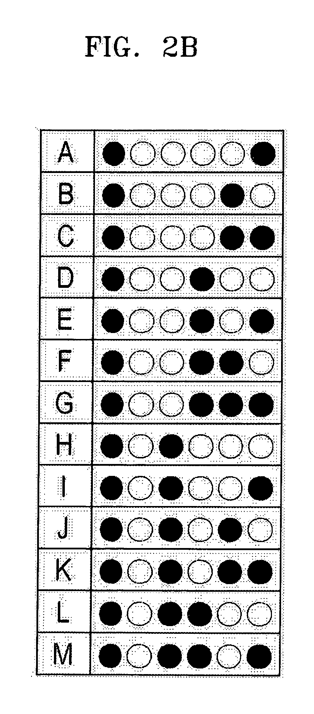

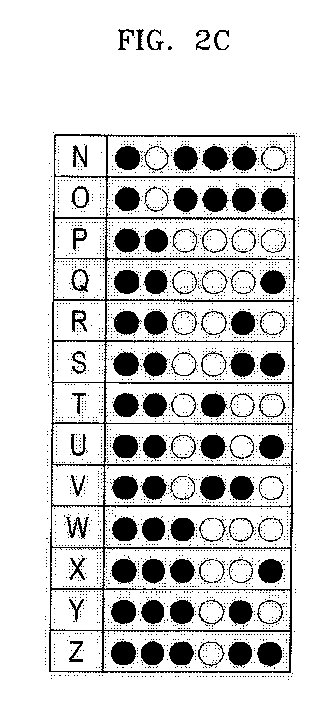

First, integers between 0 and 9, the 26 letters of the Alphabet, a discriminator for discriminating an information unit, and an "END" term designating the end of information were coded using a binary number in six orders. That is, information on each letters was transformed into a binary number expressed in the form of a spot. "Encoding table" is a table that lists the relationship of the transformation of the information into the binary number. When a microarray is produced and data from the microarray is analyzed using a computer, information on an encoding table should be stored electronically. FIG. 2A is a view illustrating a binary encoding table listing spots representing integers between 0 and 9, a discriminator for discriminating an information unit, and an "END" term designating the end of information. FIGS. 2B and 2C are views illustrating binary encoding tables listing spots representing all 26 letters of the Alphabet. In the binary encoding tables, a colored spot represents the number "1" and a vacant spot represents the number "0" . The colored spot may be coated with a detectable substance, such as Cy-3 and Cy-5, or surface-treated.

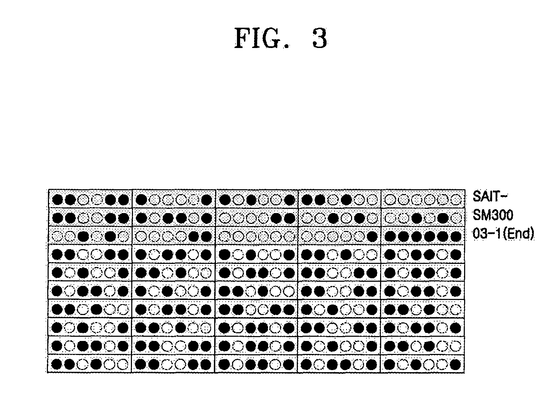

Next, a product identifier, the microarray identification information "SAIT-SM30003-1" was expressed in the form of spots using the encoding tables. The microarray identification information "SAIT-SM30003-1" corresponds to a version 1 of SM30003 manufactured by the manufacturer SAIT. FIG. 3 is a view illustrating the microarray identification information "SAIT-SM30003-1" stored in the upper 1^{st} to 3^{rd} rows of the spot region in a microarray.

Thus, the microarray identification information expressed in a binary number and the results of the experiments of a reaction of a probe substance with a target substance in an analytical method using the microarray can be simultaneously read using a detector for determining the results of the experiments of the reaction, without using a separate detector for reading microarray identification information as in the prior art, and can be together used in the following data anaylsis.

According to embodiments of the present invention, microarray identification information is stored in the form of a spot, and thus, it can be read using a detector for an analytical method using a microarray, without a need for a separate detector. In addition, the microarray identification information read by the detector can be stored in an extractable storage medium using the same detector, without a separate input, and can be used in the following analysis.

According to a method of producing a microarray in embodiments of the present invention, a microarray having microarray identification information stored in the form of a spot can be produced.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A microarray having microarray identification information stored in the form of a spot.

2. The microarray of claim 1, wherein the microarray identification information is stored using a combination of a spot having a detectable substance immobilized thereon and a spot not having a detectable substance immobilized thereon.

3. The microarray of claim 1, wherein the microarray identification information is stored using a combination of a spot having a surface subjected to a treatment so that a detectable signal can be generated therefrom and a spot having a surface not subjected to the treatment.

4. The microarray of claim 2 or 3, wherein the microarray identification information is stored in the form of a binary number or a tertiary number.

5. The microarray of claim 2 or 3, wherein one type of detectable substance is used and the microarray identification information is stored in the form of a binary number by representing a signal generated from the spot having the detectable substance immobilized thereon by 0 or 1 and a signal generated from the spot not having the detectable substance immobilized thereon by 1 or 0, in a respective order; or
one type of surface treatment is used and the microarray identification information is stored in the form of a binary number by representing a signal generated from the spot having the surface subjected to the treatment so that the detectable signal can be generated therefrom by 0 or 1 and a signal generated from the spot having the surface not subjected to the treatment by 1 or 0, in a respective order.

6. The microarray of claim 2 or 3, wherein two types of detectable substances are used and the microarray identification information is stored in the form of a tertiary number by representing a signal generated from the spot having a first detectable substance immobilized thereon by 0, 1 or 2, a signal generated from the spot having a second detectable substance immobilized thereon by 1, 2 or 0, and a signal generated from the spot not having the first or the second detectable substance immobilized thereon by 2, 0 or 1, in a respective order; or
two types of surface treatments are used and the microarray identification information is stored in the form of a tertiary number by representing a signal generated from the spot having the surface subjected to a first treatment so that the detectable signal can be generated therefrom by 0, 1 or 2, a signal generated from the spot having the surface subjected to a second treatment so that the detectable signal can be generated therefrom by 1, 2 or 0, and a signal generated from the spot having the surface not subjected to the first or the second treatment by 2, 0 or 1, in a respective order.

7. The microarray of claim 2, wherein the detectable substance is selected from the group consisting of a fluorescent substance, a radioactive substance and an enzyme capable of generating a detectable signal.

8. The microarray of claim 3, wherein the surface treatment is the formation of a grid.

9. A method of producing a microarray having microarray identification information stored in the form of a spot, comprising:
coding the microarray identification information as a number; and
immobilizing a detectable substance on a solid substrate, the coded number being expressed by using a combination of a spot having a detectable substance immobilized thereon and a spot not having a detectable substance immobilized thereon.

10. A method of producing a microarray having microarray identification information stored in the form of a spot, comprising:
coding the microarray identification information to a number; and
having a surface of a solid substrate subjected to a treatment so that a detectable signal can be generated therefrom, the coded number being expressed by using a combination of a spot having a surface subjected to the treatment and a spot having a surface not subjected to the treatment.

11. The method of claim 9 or 10, wherein the microarray identification information is stored in the form of a binary number or a tertiary number.

12. The method of claim 9 or 10, wherein one type of detectable substance is used and the microarray identification information is stored in the form of a binary number by representing a signal generated from the spot having the detectable substance immobilized thereon by 0 or 1 and a signal generated from the spot not having the detectable substance immobilized thereon by 1 or 0, in a respective order; or
one type of surface treatment is used and the microarray identification information is stored in the form of a binary number by representing a signal generated from the spot having the surface subjected to the treatment so that the detectable signal can be generated therefrom by 0 or 1 and a signal generated from the spot having the surface not subjected to the treatment by 1 or 0, in a respective order.

13. The method of claim 9 or 10, wherein two types of detectable substances are used and the microarray identification information is stored in the form of a tertiary number by representing a signal generated from the spot having a first detectable substance immobilized thereon by 0, 1 or 2, a signal generated from the spot having a second detectable substance immobilized thereon by 1, 2 or 0, and a signal generated from the spot not having the first or the second detectable substance immobilized thereon by 2, 0 or 1, in a respective order; or
two types of surface treatments are used and the microarray identification information is stored in the form of a tertiary number by representing a signal generated from the spot having the surface subjected to a first treatment so that the detectable signal can be generated therefrom by 0, 1 or 2, a signal generated from the spot having the surface subjected to a second treatment so that the detectable signal can be generated therefrom by 1, 2 or 0, and a signal generated from the spot having the surface not subjected to the first or the second treatment by 2, 0 or 1, in a respective order.

14. The method of claim 10, wherein the surface treatment is formation of a grid.
